# EUROPÄISCHE PATENTSCHRIFT

(11) **EP 3 310 393 B1**
(45) Veröffentlichungstag und Bekanntmachung des Hinweises auf die Patenterteilung: **24.07.2024**
(21) Anmeldenummer: 16733877.1
(22) Anmeldetag: 17.06.2016
(51) Int. Cl.: A61K 47/24, A61K 9/08, A61K 31/661, A61K 33/06, A61K 33/10, A61K 33/42, A61P 7/08

(54) **DIALYSELÖSUNG ODER SUBSTITUTIONSLÖSUNG ENTHALTEND EINEN ORGANISCHEN PHOSPHORSÄUREESTER**
DIALYSIS SOLUTION OR SUBSTITUTION SOLUTION COMPRISING AN ORGANIC ESTER OF PHOSPHORIC ACID
SOLUTION POUR DIALYSE OU FLUIDE DE SUBSTITUTION COMPRENANT UN ESTER ORGANIQUE DE L'ACIDE PHOSPHORIQUE

(30) Priorität: 18.06.2015 DE 102015007842
(43) Veröffentlichungstag der Anmeldung: 25.04.2018
(73) Patentinhaber: Fresenius Medical Care Deutschland GmbH, 61352 Bad Homburg (DE)
(72) Erfinder: HUPPERT, Jochen, 66132 Saarbrücken (DE); MATHIS, Pascal, 66359 Bous (DE); BERLICH, Robert, 66606 St. Wendel (DE); POHLMEIER, Robert, 61350 Bad Homburg (DE)
(74) Vertreter: Herrmann, Uwe
(86) Internationale Anmeldenummer: PCT/EP2016/001023
(87) Internationale Veröffentlichungsnummer: WO 2016/202462

(56) Entgegenhaltungen:
- EP-A1- 2 965 747
- M BROMAN ET AL: "Phosphate-containing dialysis solution prevents hypophosphatemia during continuous renal replacement therapy", ACTA ANAESTHESIOLOGICA SCANDINAVICA, 1 January 2011 (2011-01-01), England, pages 39, XP055130720, Retrieved from the Internet <URL:http://lup.lub.lu.se/record/1732502> [retrieved on 20160907], DOI: 10.1111/j.1399-6576.2010.02338.x
- TODD S ING ET AL: "REVIEW ARTICLES Phosphorus-Enriched Hemodialysates: Formulations and Clinical Use", 9 May 2003 (2003-05-09), XP055182550, Retrieved from the Internet <URL:http://onlinelibrary.wiley.com/store/10.1046/j.1492-7535.2003.00023.x/asset/j.1492-7535.2003.00023.x.pdf?v=1&t=i8fwft1a&s=3df20eb20088d0784077c5e8e5627c9a8ef517af> [retrieved on 20150413]

## Beschreibung

Die vorliegende Erfindung betrifft eine Bicarbonat-gepufferte Dialyse- oder Substitutionslösung, die Calcium- und/oder Magnesium-Ionen sowie Glycerin-ortho-phosphat und ortho-Phosphat enthält. Weitere Merkmale sind in den Ansprüchen definiert. Die vorliegende Erfindung betrifft darüber hinaus eine Kombination aus mehreren, vorzugsweise aus genau zwei Einzellösungen in einem Mehrkammerbeutel, die nach ihrem Mischen eine wie vorstehend beschriebene Dialyselösung oder Substitutionslösung ergeben.

Die Erfindung wird durch die Ansprüche definiert. Jegliche Gegenstände, die nicht unter die Ansprüche fallen, werden lediglich zu Referenz- oder Vergleichszwecken offenbart.

Calcium- oder Magnesium-ionenhaltige, Bicarbonat-gepufferte Dialyselösungen enthalten üblicherweise Elektrolyte, Puffer und Glucose in physiologisch wirksamen Konzentrationen. Ein Problem bei Dialyselösungen, die neben Calcium oder Magnesium auch Bicarbonat als Puffer enthalten, besteht darin, dass unter bestimmten Bedingungen, insbesondere bei einem vergleichsweise hohen pH-Wert und höheren Temperaturen schwerlösliche Carbonate gebildet werden können, was unerwünscht ist.

Dialyselösungen mit einem physiologischen Phosphatgehalt werden in der Akutdialyse dazu eingesetzt, um den Phosphathaushalt von Patienten zu regulieren und einer Hypophosphatämie vorzubeugen. Eine medizinisch sinnvolle Phosphatkonzentration liegt im Bereich von 0,60 bis 1,45 mmol/L, vorzugsweise 0,8 bis 1,25 mmol/l basierend auf Erfahrungen aus der klinischen Anwendung.

Enthält eine Calcium- oder Magnesium-Ionenhaltige, Bicarbonat-gepufferte Dialyselösung zusätzlich zu den Bicarbonat-Ionen noch Phosphat-Ionen, so ist potentiell auch die Gefahr gegeben, dass sich schwerlösliche Phosphatverbindungen ausbilden können. Aufgrund Ihrer geringen Löslichkeit sind Erdalkaliphosphat-Ausfällungen medizinisch gesehen noch kritischer einzustufen als Erdalkalicarbonat-Ausfällungen und sollten daher unbedingt vermieden werden.

Insbesondere ist eine pH-Wert Erhöhung bedingt durch einen Verlust an CO₂ durch Ausgasen für die Erdalkalicarbonat- und Erdalkaliphosphat-Ausfällungen verantwortlich. Unter thermodynamischen Gesichtspunkten gibt es einen maximalen pH-Wert, bis zu dem die Dialyselösung stabil bleibt, d.h. bis zu dem die genannten Ausfällungen nicht auftreten. Steigt der pH-Wert der Dialyselösung unter Anwendungsbedingungen an, wie beispielsweise durch das Pumpen und Heizen an einer Dialysemaschine oder durch Lagerung, kann ein metastabiler Zustand erreicht werden. Kollabiert dieser Zustand, fallen schwerlösliche Carbonate und/oder Phosphate aus, was zu erheblichen Komplikationen bei der Behandlung führen kann. Schwerlösliche Magnesiumphosphate und Calciumphosphate stellen hierbei aufgrund der geringen Löslichkeit im basischen Milieu die kritischsten Verbindungen dar. Aber auch Magnesiumcarbonat und Calciumcarbonat stellen aufgrund der schlechten Löslichkeit im basischen Milieu kritische Verbindungen dar.

Aus dem Stand der Technik ist es bekannt, Calcium- oder Magnesium-lonen-haltige, Bicarbonat-gepufferte Dialyselösungen in Form von Einzellösungen bereitzustellen, die in einem Doppelkammer-Beutel aufgenommen sind. Dies lässt sich auch für Lösungen realisieren, die ferner phosphathaltig sind. Eine anwendungsfertige Dialyselösung wird durch das Mischen der beiden Kammerinhalte erhalten. Durch die Bereitstellung der Einzellösungen in einem Doppelkammerbeutel kann eine getrennte Lagerung von Calcium einerseits und Bicarbonat bzw. Phosphat andererseits und somit eine erhöhte Stabilität bei der Lagerung der Dialyselösung erreicht werden. Aus dem Stand der Technik ist es weiter bekannt, die Beutelfolie aus einer Barrierefolie herzustellen, um dem Entweichen von CO₂ und somit der Erhöhung des pH-Wertes in der Bicarbonat- und ggf. Phosphat-haltigen Einzellösung entgegenzuwirken. Gleichwohl steigt trotzt dieser speziellen Verpackung der pH-Wert der bicarbonat- und ggf. phosphathaltigen Dialyselösung über die Lagerdauer an, was zur Folge hat, dass beim Mischen der beiden Einzellösungen der pH-Wert der Mischlösung, d.h. der fertigen Dialyselösung vor deren Gebrauch ebenfalls erhöht ist. Um Ausfällungen beim Mischen oder bei der Anwendung an der Dialysemaschine zu vermeiden, muss sichergestellt sein, dass der pH-Wert der Bicarbonat- und ggf. Phosphat-haltigen Dialyselösung sowie der pH-Wert der aus den Einzellösungen hergestellten Mischung in einem relativ engen Rahmen liegen.

Relevanter Stand der Technik ist beispielsweise M Broman et al.: Phosphatecontaining dialysis solution prevents hypophosphatemia during continuous renal replacement therapy", Acta anaesthesiologica Scandinavica, 1. Januar 2011 (2011-01-01), Seite 39, XP055130720, England DOI:10.1111/j.1399-6576.2010.012338.x. Weiterhin offenbart die Druckschrift EP2965747A1 eine Zusammensetzung zur Erzeugung von Dialysierflüssigkeit.

Darüber hinaus befasst sich die Druckschrift Todd S Ing et al: "Review Articles Phosphorus-Enriched Hemodialysates: Formulations and Clinical Use", 9. Mai 2003 (2003-05-09), XP055182550 mit der Thematik von Phosphaten in Dialysierflüssigkeiten.

Der vorliegenden Erfindung liegt somit die Aufgabe zugrunde, eine Calcium- oder Magnesium-lonen-haltige, Bicarbonat-gepufferte Dialyse- oder Substitutionslösung, welche einen vorzugsweise physiologischen Phosphatgehalt aufweisen soll, dahingehend weiterzubilden, dass die Wahrscheinlichkeit für das Auftreten von Ausfällungen gegenüber bekannten Lösungen verringert ist.

Diese Aufgabe wird durch eine Dialyselösung oder Substitutionslösung mit den Merkmalen des Anspruchs 1 gelöst. Eine erfindungsgemäße Dialyselösung oder Substitutionslösung enthält demnach Bicarbonat-Ionen sowie Calcium- und/oder Magnesium-Ionen, wobei ferner vorgesehen ist, dass die Lösung Glycerin-ortho-phosphat enthält.

Es hat sich gezeigt, dass derartige organische Ester eine Phosphatquelle darstellen können, die einerseits vom Körper schnell aufgenommen wird, und andererseits keine schwerlöslichen Phosphate ausbildet und die Lösung sogar darüber hinaus stabilisiert. Die zusätzliche Stabilisierung der Dialyselösung oder Substitutionslösung bzw. von Einzellösungen, aus denen die Dialyselösung oder Substitutionslösung erhalten wird, ist vermutlich darauf zurückzuführen, das Kristallwachstum von Calcium- und/oder Magnesiumcarbonat in einer gattungsgemäßen Dialyselösung oder Substitutionslösung durch das Vorhandensein des organischen Esters der Phosphorsäure verlangsamt oder sogar komplett inhibiert wird. Die obere pH-Wert Grenze, bei der eine Ausfällung von Carbonat erfolgt, wird weiter ins basische Milieu, d.h. zu höheren pH-Werten hin verschoben. Etwaige Ausfällungsreaktionen finden dann erst bei so hohen pH-Werten statt, die während der Dialysebehandlung bzw. während der Lagerung der Dialyselösung oder Substitutionslösung - ggf. in Einzellösungen aufgeteilt - üblicherweise nicht erreicht werden. So kann eine anwendungssichere Dialyselösung oder Substitutionslösung über die komplette

Haltbarkeitsdauer des Produktes, vorzugsweise über eine Dauer von 24 Monaten oder länger gewährleistet werden. Ferner führt dies zu einem erheblichen Sicherheitsgewinn bei Anwendung der Dialyselösung oder Substitutionslösung an einer Dialysemaschine.

Die Begriffe "*Dialyselösung*" und "*Substitutionslösung*" umfasst vorliegend sowohl Konzentrate, die vor der Verwendung weiter verdünnt werden müssen, als auch anwendungsfertige Lösungen, die als solche im Rahmen der Dialyse verwendet werden können. Umfasst sind sowohl Dialyse- oder Substitutionslösungen für die Hämodialyse, Hämodiafiltration und Hämofiltration als auch Lösungen für die Peritonealdialyse.

Die Nennung des Begriffs "*Dialyselösung"* umfasst im Rahmen der vorliegenden Erfindung auch eine Substitutionslösung und umgekehrt. Beide Begriffe stellen somit jeweils Platzhalter sowohl für eine Dialyselösung als auch für eine Substitutionslösung dar.

Bei einem organischen Ester der Phosphorsäure kann es sich beispielsweise um einen organischen Ester der ortho-Phosphorsäure, vorzugsweise einen organischen Monoester der ortho-Phosphorsäure handeln.

Anspruchsgemäß handelt es sich bei dem organischen Ester der *ortho-*Phosphorsäure um Glycerin-*ortho*-phosphat. Dieser Stoff ist bereits als Wirkstoff beispielsweise bei der parenteralen Ernährung etabliert und auch im Europäischen Arzneibuch monographiert (01/2009:1995). Dieses relativ kleine Molekül kann schnell unter Freisetzung von ortho-Phosphat verstoffwechselt werden. Das Glycerin-ortho-phosphat kann ein Glycerin-2-ortho-phosphat, ein Glycerin-3-ortho-phosphat oder eine Mischung daraus sein.

Die Dialyselösung oder Substitutionslösung enthält veresterte und nicht veresterte Phosphatgruppen in einer Konzentration von 0,8 bis 1,25 mmol/l und vorzugsweise von 1 bis 1,2 mmol/l. Eine Phosphat-Konzentration von 0,8 bis 1,25 mmol/l und vorzugsweise von 1 bis 1,2 mmol/l entspricht einer Konzentration, die dazu verwendet werden kann, den Phosphathaushalt von Dialysepatienten zu regulieren und beispielsweise einer Hypophosphatämie vorzubeugen. So wird durch die Zugabe des organischen Esters der Phosphorsäure nicht nur die Lösung stabilisiert sondern auch eine wünschenswerte physiologische Wirkung erzielt.

Die Dialyselösung oder Substitutionslösung enthält zusätzlich zum organischen Ester der Phosphorsäure des Weiteren ortho-Phosphat. Es hat sich gezeigt, dass bei Zugabe von sowohl einem organischen Ester der Phosphorsäure als auch von ortho-Phosphat ein synergischer Effekt auftritt, der zu einer noch besseren Stabilisierung der Lösung gegenüber der Ausfällung von Calciumcarbonat führt.

Zusätzlich zum organischen Ester der Phosphorsäure ist auch *ortho*-Phosphat in einer Konzentration von >0 bis 0,3 mmol/l und vorzugsweise von 0,1 bis 0,2 mmol/l enthalten. In diesen Konzentrationsbereichen ist der synergische Effekt besonders stark zu beobachten und der Gesamtgehalt des Phosphats bewegt sich zudem im physiologischen Bereich.

Die Dialyselösung oder die Substitutionslösung enthält veresterte und nicht veresterte Phosphorgruppen in der Summe in einer Konzentration von 0,8 bis 1,25 mmol/l und vorzugsweise von 1 bis 1,2 mmol/l.

Die Dialyselösung oder Substitutionslösung enthält neben den Calcium- und/oder Magnesium-Ionen Natrium-Ionen, Kalium-Ionen und/oder Chlorid-Ionen als weitere Elektrolyte.

In einer Ausführungsform enthält die Dialyselösung oder Substitutionslösung ferner wenigstens ein Osmotikum, vorzugsweise ein Saccharid, vorzugsweise Glukose.

In der Dialyselösung oder Substitutionslösung können die genannten Solvate unabhängig voneinander beispielsweise in den folgenden Konzentrationen vorliegen:

**Tabelle 1**

| | |
|---|---|
| Calcium-Ionen: | 1-2 mmol/l, beispielsweise 1,5 mmol/l |
| Magnesium-Ionen: | 0,2-0,8 mmol/l, beispielsweise 0,5 oder 0,75 mmol/l |
| Kalium-Ionen: | bis zu 8 und vorzugsweise bis zu 4 mmol/l |
| Natrium-Ionen: | 120-160 mmol/l, beispielsweise 140 mmol/l |
| Bicarbonat-Ionen (inkl. Carbonat-Ionen und gelöstem CO₂): | 30-40 mmol/l, beispielsweise 35 mmol/l |
| Osmotikum: | 4-12 mmol/l, beispielsweise 5,6 mmol/l |
| Chlorid-Ionen: | 100-120 mmol/l, beispielsweise 109 mmol/l |

In einer Ausführungsform liegt der pH-Wert der Dialyselösung oder Substitutionslösung im Bereich von 7,0 bis 7,6.

Die Erfindung betrifft des Weiteren eine Kombination aus mehreren, vorzugsweise aus genau zwei Einzellösungen, die derart ausgebildet sind, dass sie nach ihrem Mischen miteinander eine erfindungsgemäße Dialyselösung oder Substitutionslösung ausbilden.

In einer Ausführungsform ist dabei vorgesehen, dass nur eine der Einzellösungen den organischen Ester der Phosphorsäure und ggf. die ortho-Phosphorsäure enthält.

Beispielsweise kann vorgesehen sein, dass eine erste Einzellösung Calcium-und/oder Magnesium-lonen enthält und eine zweite Einzellösung, die keine Calcium-und/oder Magnesium-lonen enthält, den organischen Ester der Phosphorsäure und ggf. die *ortho*-Phosphorsäure enthält.

Des Weiteren kann beispielsweise vorgesehen sein, dass eine erste Einzellösung Calcium- und/oder Magnesium-Ionen, Chlorid-lonen, ein Osmotikum und ggf. Kalium-Ionen enthält und eine zweite Einzellösung Natrium-Ionen, Chlorid-Ionen, Bicarbonat-Ionen, den organischen Ester der Phosphorsäure und ggf. die *ortho-*Phosphorsäure enthält.

In einer Ausführungsform enthält die erste Einzellösung keine Bicarbonat-Ionen und/oder keinen organischen Ester der Phosphorsäure und/oder keine *ortho-*Phosphorsäure und/oder keine Natrium-Ionen.

In einer Ausführungsform enthält die zweite Einzellösung keine Calcium- und/oder Magnesium-Ionen und/oder keine Kalium-Ionen und/oder kein Osmotikum.

In der jeweiligen Einzellösung können die genannten Solvate unabhängig voneinander beispielsweise in den folgenden Konzentrationen vorliegen:

**Tabelle 2**

| | |
|---|---|
| Calcium-Ionen: | 20-40 mmol/l, beispielsweise 30 mmol/l |
| Magnesium-Ionen: | 5-15 mmol/l, beispielsweise 10 mmol/l |
| Kalium-Ionen: | bis zu 100 mmol/l |
| Natrium-Ionen: | 100-200 mmol/l, beispielsweise 140-160 mmol/l oder genau 147,5 mmol/l |
| Bicarbonat- Ionen (inkl. Carbonat-Ionen und gelöstem CO₂): | 30-50 mmol/l, beispielsweise 37 mmol/l |
| Osmotikum: | 100-250 mmol/l, beispielsweise 111 mmol/l |
| Chlorid-Ionen: | 60-100 mmol/l oder 100-120 mmol/l |
| Organischer Ester der Phosphorsäure (bezogen auf das Phosphat) | 0,8-1,25 mmol/l |
| Ortho-Phosphorsäure (sofern vorhanden) | >0-0,3 mmol/l, beispielsweise 0,1-0,2 mmol/l |

Chlorid-lonen können beispielsweise in beiden Einzellösungen vorliegen. Dabei kann die Chlorid-Ionen-Konzentration in einer Einzellösung, die das Osmotikum enthält, in einem Konzentrationsbereich von 60 mmol/l bis 100 mmol/l und beispielsweise bei genau 82 mmol/l liegen und in einer Einzellösung, die den Puffer und/oder den organischen Ester der Phosphorsäure enthält in einem Konzentrationsbereich von 100 mmol/l bis 120 mmol/l und beispielsweise bei genau 110 mmol/l liegen.

In einer Ausführungsform weist eine erste Einzellösung einen pH-Wert im Bereich von 2,4 bis 3,0 auf und eine zweite, den organischen Ester der Phosphorsäure enthaltende Einzellösung einen pH-Wert im Bereich von 7,0 bis 7,8 auf.

In einer Ausführungsform ist vorgesehen, dass die Kombination zwei Einzellösungen A und B aufweist, wobei die Lösung A ein, mehrere oder alle vorgenannten Charakteristika einer ersten Einzellösung aufweist und/oder wobei die Lösung B ein, mehrere oder alle vorgenannten Charakteristika einer zweiten Einzellösung aufweist.

Letztlich betrifft die Erfindung auch einen Mehrkammerbeutel umfassend wenigstens zwei Kammern, wobei eine der Kammern eine erste Einzellösung der erfindungsgemäßen Kombination und eine andere Kammer eine zweite Einzellösung der erfindungsgemäßen Kombination aufweist. Der Mehrkammerbeutel kann wenigstens ein Trennmittel aufweisen das unterschiedliche Kammern voneinander trennt. Bei dem Trennmittel kann es sich beispielsweise um eine Schweißnaht handeln. Das Trennmittel bzw. die Schweißnaht ist vorzugsweise so ausgebildet, dass es bzw. sie durch Druck auf eine der Kammern so geöffnet wird, dass eine Verbindung zwischen den getrennten Kammern entsteht.

Weitere Einzelheiten und Vorteile der Erfindung ergeben sich aus den nachfolgend auch anhand der Figuren erläuterten Ausführungsbeispiele und Vergleichsbeispiele. In den Figuren zeigen:
- Figur 1:: eine schematische Darstellung sicherer und kritischer pH-Bereiche einer Bicarbonat-gepufferte Lösung aus dem Stand der Technik;
- Figur 2:: eine schematische Darstellung sicherer und kritischer pH-Bereiche einer Bicarbonat-gepufferte Lösung mit verbesserter Stabilität;
- Figur 3:: schematische Darstellungen zur analytischen Detektion einer Fällung in Dialyselösungen;
- Figur 4:: eine Darstellung des pH-Wertes einer Dialyselösung im zeitlichen Verlauf eines Ausgasexperiments;
- Figur 5:: eine Darstellung der Trübung zweier Dialyselösungen im zeitlichen Verlauf eines Ausgasexperiments;
- Figur 6:: eine Darstellung des pH-Wertes einer phosphathaltigen Dialyselösung im zeitlichen Verlauf eines Ausgasexperiments;
- Figur 7:: eine Darstellung des pH-Wertes einer phosphatfreien Dialyselösung im zeitlichen Verlauf eines Ausgasexperiments;
- Figur 8:: EDX-Spektrum des isolierten Fällungsprodukts einer phosphathaltigen Dialyselösung;
- Figur 9:: Strukturformeln von Glycerin-2-ortho-Phosphat und Glycerin-3-ortho-Phosphat;
- Figur 10:: eine Darstellung der pHₘₐₓ- und t_{G}-Werte von Dialyselösungen mit unterschiedlichen Konzentrationen an Glycerin-2-ortho-Phosphat;
- Figur 11:: eine Darstellung der pH-Werte zweier Dialyselösungen im zeitlichen Verlauf eines Ausgasexperiments;
- Figur 12:: eine Darstellung der pH-Werte dreier Dialyselösungen im zeitlichen Verlauf eines Ausgasexperiments;
- Figur 13,: eine Darstellung der pHₘₐₓ- und t_{G}-Werte von Dialyselösungen mit unterschiedlichen Konzentrationen an Glycerin-2-ortho-Phosphat und/oder ortho-Phosphat; und
- Figur 14:: eine Darstellung der pHₘₐₓ- und t_{G}-Werte von Dialyselösungen mit unterschiedlichen Konzentrationen an Glycerin-2-ortho-Phosphat und ortho-Phosphat mit einer konstanten Gesamtkonzentration beider Stoffe sowie der MultiBic- und einer ortho-Phosphat haltigen Dialyselösung, jeweils ohne Glycerin-2-ortho-Phosphat; und
- Figur 15:: eine Darstellung der pHₘₐₓ- und t_{G}-Werte von Dialyselösungen mit Glycerin-2-ortho-Phosphat und unterschiedlichen Konzentrationen an ortho-Phosphat.

### Schematische Erklärung der Erhöhung der Stabilität

Ein maßgeblicher Vorteil der vorliegenden Erfindung ist eine Erhöhung der Stabilität der Dialyselösung gegenüber Ausfällungen im Vergleich zu bekannten gattungsgemäßen Dialyselösungen mit vergleichbaren physiologischen Effekten.

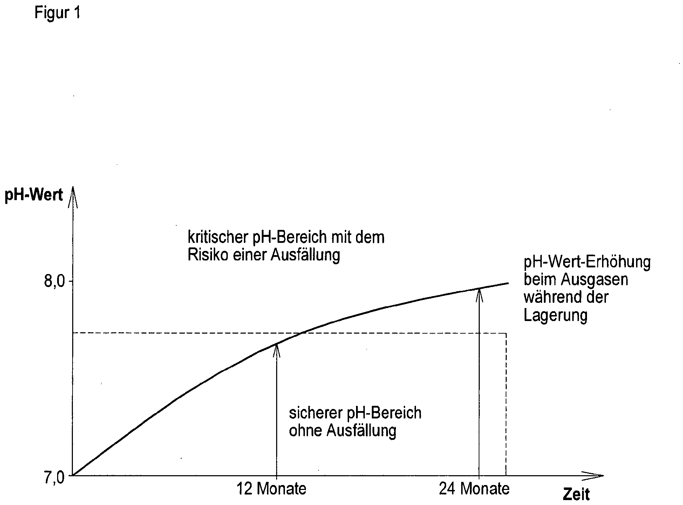

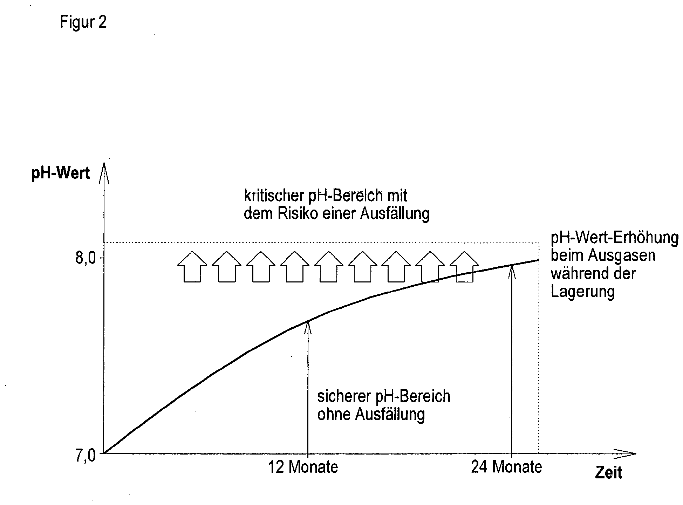

Dieser Effekt ist in den schematischen Darstellungen sicherer und kritischer pH-Bereiche gemäß Figuren 1 und 2 dargestellt. Figur 1 illustriert die entsprechenden Bereiche für eine Bicarbonat-gepufferte Lösung aus dem Stand der Technik. Figur 2 illustriert die entsprechenden Bereiche für eine Bicarbonat-gepufferte Lösung mit verbesserter Stabilität.

Die Kennlinie in Figur 1 zeigt den Verlauf des pH-Wertes über die Lagerdauer. Unterhalb eines pH-Wertes von ca. 7,5 befindet sich ein sicherer Bereich, in dem für bei einer Bicarbonat-gepufferten Lösung aus dem Stand der Technik keine Ausfällung von Calcium-lonen oder Magnesium-Ionen als Carbonat oder Phosphat stattfindet. Oberhalb eines pH-Wertes von ca. 7,5 befindet sich ein kritischer Bereich, in dem bei einer Bicarbonat-gepufferten Lösung aus dem Stand der Technik eine derartige Ausfällung stattfindet, sobald der metastabile Zustand kollabiert.

Der sichere Bereich wird in einer Bicarbonat-gepufferte Lösung mit verbesserter Stabilität (Figur 2) gegenüber dem Stand der Technik (Figur 1) zu höheren pH-Werten erweitert. Während daher die Lösung aus dem Stand der Technik nach einer bestimmten Lagerdauer (im vorliegenden Beispiel etwa 12 Monate) den sicheren Bereich verlässt (Figur 1), verweilt die stabilisierte Lösung wesentlich länger (im vorliegenden Beispiel mehr als 24 Monate) im sicheren Bereich.

### Bestimmung der Stabilität

Zur Bestimmung der Stabilität der Dialyselösung kann beispielsweise die "Rapid Controlled Precipitation Method" oder die "Critical pH-method" herangezogen werden, wie sie in F. Hui et al: Journal European of Water Quality (Journal Europeen d'Hydrologie) T.33 Fase. 1 (2002) beschrieben ist.

Die im Rahmen dieser Erfindung beschriebenen Ergebnisse wurden durch eine modifizierte Rapid Controlled Precipitation Method erhalten. Der Versuchsaufbau besteht aus sechs 3-Halskolben (Carousel-6 der Fa. Radleys), die nach oben hin geöffnet sind, um eine gleichmäßige Ausgasung von CO₂ aus der Lösung zu gewährleisten. Weiterhin erlaubt dieser Aufbau eine in-line Messung von z.B. pH-Wert und Leitfähigkeit sowie das simultane Heizen der Kolben.

Das Grundprinzip der verwendeten Methode besteht darin, dass der pH-Wert der Mischlösung bzw. der Dialyselösung durch kontrollierte Ausgasung von CO₂ langsam angehoben wird, bis die Dialyselösung einen metastabilen Zustand erreicht und letztlich ausfällt.

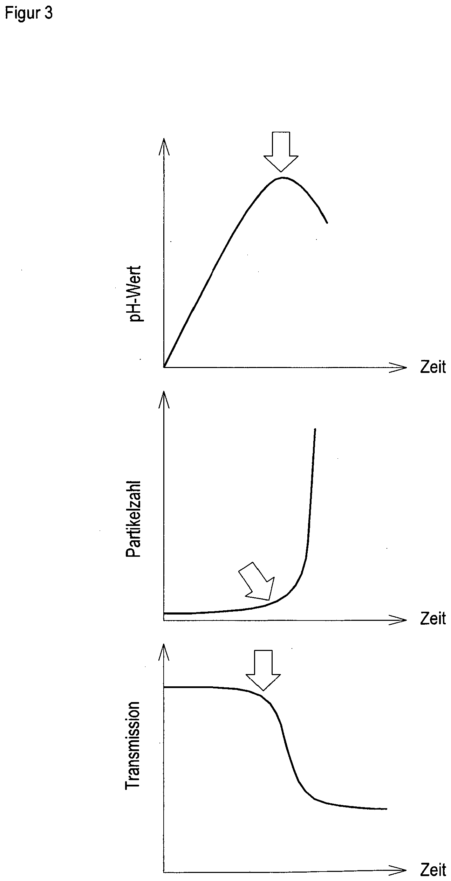

Als Methoden zur Detektion der Ausfällung kann beispielsweise eine pH-Wert-Messung, eine Partikelzählung oder eine Trübungsmessung herangezogen werden. Es empfiehlt sich hierbei eine in-line Messung durchzuführen, um den genauen Zeitpunkt des Eintretens der Fällung scharf zu detektieren und nicht durch eine Probenaufbereitung und -transport zum Analysegerät zu verfälschen. Die anhand dieser Methoden erhältlichen Messkurven sind in Figur 3 gegenübergestellt.

Im Falle der pH-Messung kann der Beginn der Carbonat-Fällung an einem signifikanten Abknicken des pH-Wertes erkannt werden (Figur 3, linke Darstellung). Über die Zeit gesehen steigt der pH-Wert durch das Ausgasen von CO₂ an und erreicht ein Maximum (pHₘₐₓ), an dem die Fällungsreaktion beginnt. In vielen Fällen kann dieser pHₘₐₓ-Wert als Kriterium für die Stabilität einer Dialyselösung herangezogen werden. Bei der Partikelmessung erkennt man den Beginn der Fällung an einem Ansteigen der Partikelzahl (Figur 3, mittlere Darstellung), bei der Trübungsmessung an einem Abfall der Transmission bzw. Lichtdurchlässigkeit (Figur 3, rechte Darstellung). Der Zeitpunkt des Fällungsbeginns wird als t_{G} (time of germination) bezeichnet.

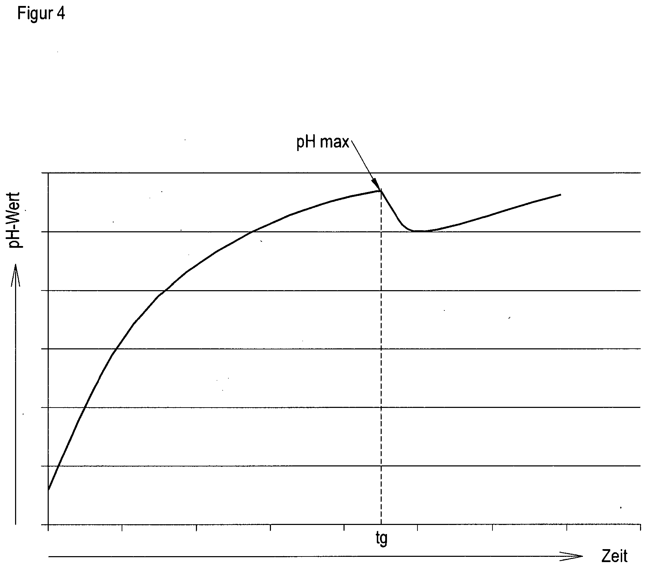

Je höher der pHₘₐₓ-Wert, umso höher ist auch die Stabilität der Lösung. Eine höhere Stabilität bedeutet auch größere t_{G}-Zeiten unter gleichen Ausgasungsbedingungen. Die Kenngrößen bzgl. der Lösungsstabilität sind in Figur 4 anschaulich am Beispiel eines Ausgasungsversuchs mit Calciumcarbonat-Ausfällung dargestellt.

In Figur 4 lässt sich die Erhöhung des pH-Wertes bis zum Zeitpunkt t_{G} durch das Ausgasen von CO₂ aus der Dialyselösung erklären. Wie dies weiter aus Figur 4 ersichtlich ist, entsteht ein lokales pH-Wertmaximum. Nach diesem Punkt tritt Übersättigung der Dialyselösung ein und es kommt zur Ausfällung von Calciumcarbonat. Bei der Ausfällung werden der Dialyselösung Carbonat-Ionen entzogen. Aufgrund der Gleichgewichtsreaktion mit Bicarbonat werden vermehrt Protonen gebildet, was zu der Abnahme des pH-Wertes führt.

Durch den Zusatz eines organischen Esters der Phosphorsäure kann die Stabilität der Dialyselösung oder einer Einzellösung signifikant erhöht werden, wobei das Zusammenbrechen des metastabilen Bereiches verzögert oder ganz verhindert wird.

### Vergleichsbeispiel

Zu einer Bicarbonat-gepufferten Dialyselösung, die Calcium- und/oder Magnesium-Ionen enthält, wird ortho-Phosphat in einer physiologischen Konzentration zugegeben.

Gibt man zu einer herkömmlichen "*multiBic"* 0K Lösung der Fresenius Medical Care Deutschland GmbH 1,0 mmol/l *ortho*-Phosphat unter Beibehaltung der restlichen Lösungsbestandteile, so ist aus dem Vergleich der unmodifizierten und der *ortho-*Phosphat-("*P*")-modifizierten Lösungen erkennbar, dass die "*multiBic*"-Lösung eine t_{G}-Zeit von ca. 2 Stunden erreicht. Die Zugabe von 1,0 mmol/l *ortho*-Phosphat führt zu einer Stabilisierung der Lösung und die t_{G}-Zeiten erhöht sich auf knapp über 6,5 Stunden.

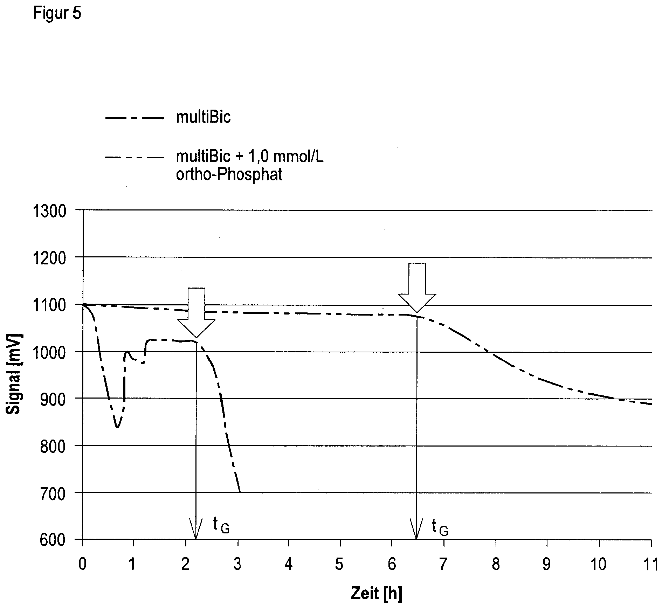

Figur 5 zeigt die entsprechenden Messkurven, die anhand der Rapid Controlled Precipitation Method bei T=40°C mit Trübungsmessung als Detektion erhalten wurden.

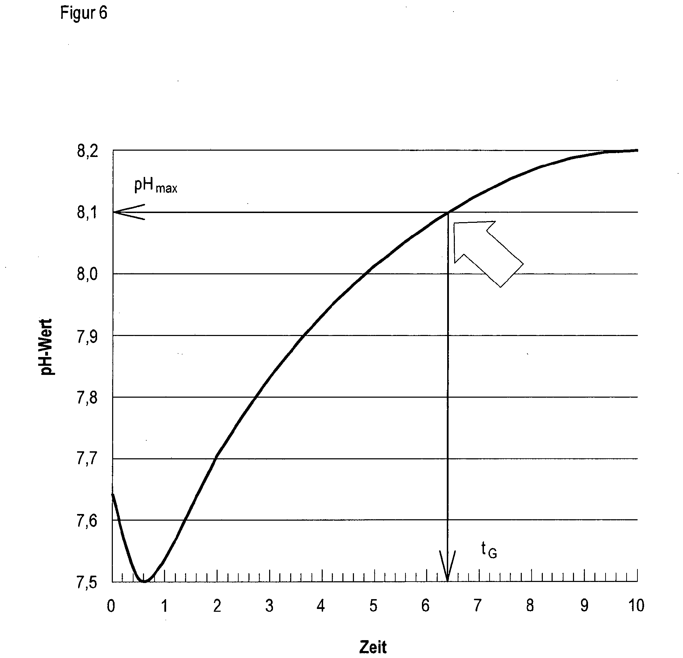

Interessanterweise zeigt die pH-Messkurve einer entsprechenden Messung an der P-modifizierten "*multiBic*"*-*Lösung, die in Figur 6 dargestellt ist, keinen signifikanten Knick und ist deshalb nicht geeignet, um die Fällung zu detektieren. Die pHₘₐₓ-Werte mussten in diesem Fall daher aus den mittels Partikel-oder Trübungsmessung bestimmten t_{G}-Werten abgeleitet werden.

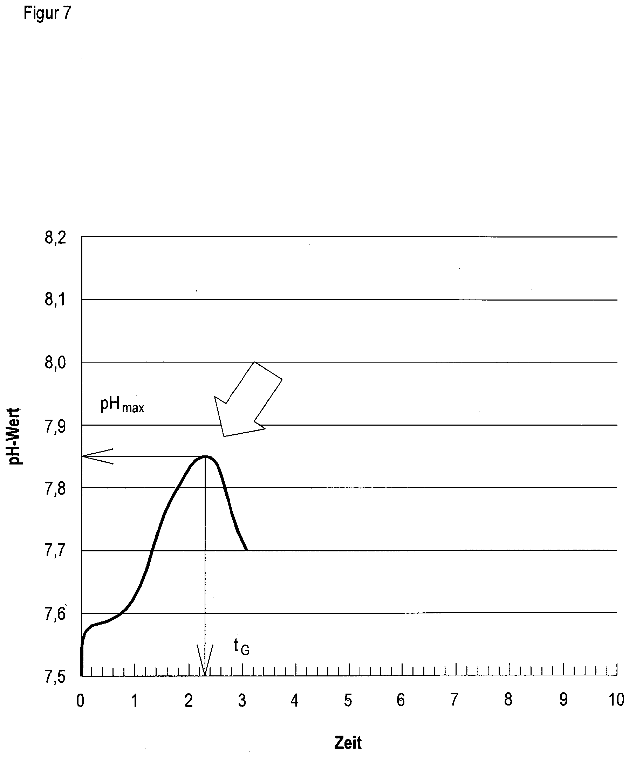

Die pH-Messkurve der phosphatfreien unmodifizierten *"multiBic"-Lösung,* die in Figur 7 dargestellt ist, zeigt hingegen das gewohnte Bild des signifikanten Abfalls beim Eintritt der Fällung. Der pHₘₐₓ-Wert liegt hier bei 7,85. Der t_{G}-Wert stimmt gut mit dem Wert aus der Trübungsmessung überein.

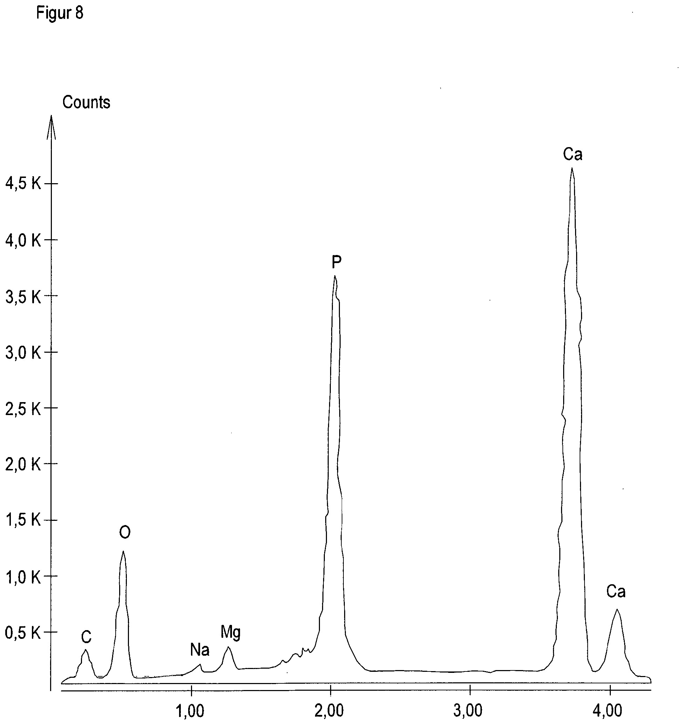

Dieses Verhalten der P-modifizierten *"multiBic"-Lösung* deutet darauf hin, dass sich eine andere schwerlösliche Phase bildet. Diese Annahme wird durch ein in Figur 8 gezeigtes EDX-Spektrum bestätigt, das für die OP-modifizierte "*multiBic*"-Lösung mit physiologischer Phosphat-Konzentration klar zeigt, dass als Hauptprodukt eine Calciumphosphatverbindung entsteht.

Bei dem Niederschlag könnte es sich beispielsweise um ein Calciumhydrogenphosphat handeln, welches sich gemäß folgender Gleichung bildet:

Ca²⁺ + HPO₄²⁻ → CaHPO₄ ↓

Da bei dieser Reaktion keine Protonen freigesetzt werden, ist auch kein Abfall des pH-Signals zu erkennen, was das Fehlen des signifikanten pH-Knicks erklärt.

Die Ausfällung von Calciumcarbonat hingegen setzt Protonen frei, die Fällung kann in diesem Fall mittels pH-Messung detektiert werden:

Ca²⁺ + HCO₃⁻ → CaCO₃ ↓ + H⁺

Die Zusammensetzungen und pH-Werte der unmodifizierten "*multiBic*"-Lösung, der P-modifizierten "*multiBic*"-Lösung, einer herkömmlichen Phosphat-haltigen *"multiPlus"-Lösung* von Fresenius Medical Care und einer herkömmlichen Phosphat-haltigen *"Phoxilium"-Lösung* von Gambro sind in der nachfolgenden Tabelle 3 dargestellt (bei den angegebenen Werten handelt es sich um Hestellerangaben):

**Tabelle 3:**

| | unmodifizierte *"multiBic"-Lösung* | P-modifizierte "*multiBic*"-Lösung | "*multiPlus*"-Lösung | *"Phoxilium"-*Lösung |
|---|---|---|---|---|
| Na⁺ | 140 | 140 | 140 | 140 |
| K⁺ | 0 | 0 | 2 | 4 |
| Mg²⁺ | 0,50 | 0,50 | 0,75 | 0,60 |
| Ca²⁺ | 1,50 | 1,50 | 1,50 | 1,25 |
| Cl⁻ | 109,0 | 109,0 | 110,5 | 115,9 |
| HCO₃⁻ | 35 | 35 | 35 | 30 |
| HPO₄²⁻ | 0 | 1,0 | 1,0 | 1,2 |
| Glucose | 5,55 | 5,55 | 5,55 | 0 |
| pH-Wert | ~7,4 | ~7,4 | ~7,4 | ~7,4 |

Die *"Phoxilium"-Lösung* enthält mit 1,20 mmol/l die höchste Phosphat-Konzentration, jedoch keine Glucose und die niedrigsten Konzentrationen an Calcium und Bicarbonat. Des Weiteren ist die Magnesium-Konzentration bei der *"Phoxilium"-*Lösung und der *"multiPlus"-Lösung* erhöht.

In der nachfolgend wiedergegebenen Tabelle 4 sind der pHₘₐₓ sowie die Ausfällungsprodukte der unterschiedlichen Lösungen zusammengestellt. Die Werte wurden alle bei 40°C mit der "*Rapid* Degassing"-Methode bestimmt.

**Tabelle 4:**

| | pHₘₐₓ | Ausfällungsprodukt |
|---|---|---|
| unmodifizierte "*multiBic*"*-*Lösung | 7,9 | Calciumcarbonat |
| P-modifizierte "*multiBic*"-Lösung | 8,3* | Calciumphosphat |
| "*multiPlus*"*-*Lösung | 8,3* | Calciumphosphat |
| *"Phoxilium"-Lösung* | 8,2* | Calciumphosphat |

| | | |
|---|---|---|
| * abgeleitet aus Trübungs- und/oder Partikelmessung | | |

Die Stabilitätsmessungen zeigen, dass die Anwesenheit von ortho-Phosphat die Stabilität der Lösung gegenüber Ausfällungsreaktion erhöht, jedoch den entscheidenden Nachteil hat, dass immer Calciumphosphat als Fällungsprodukt auftritt. Sollten bei einer Behandlung mit diesen Phosphat-haltigen Lösungen trotz aller Vorsichtsmaßnahmen Partikel ausfallen und infundiert werden, so hätte dies vermutlich schwerwiegendere Folgen als bei einer reinen Bicarbonat-haltigen Lösung ohne Phosphatzusatz.

Des Weiteren hat z.B. die spezielle Zusammensetzung der *"Phoxilium"-Lösung* den Nachteil, dass die geringeren Calcium- und Bicarbonat-Konzentrationen sich in einer CRRT-Behandlung negativ äußern können, wodurch bei den Patienten Hypokalzämie und Azidose auftreten können (Journal of Critical Care, 28, 5, 2013, 884.e7-884.e14).

### Ausführungsbeispiel 1

Die vorliegende Erfindung schlägt Phosphatquellen vor, die vom Körper schnell aufgenommen werden, die Lösung stabilisieren und keine schwerlöslichen Phosphate ausbilden.

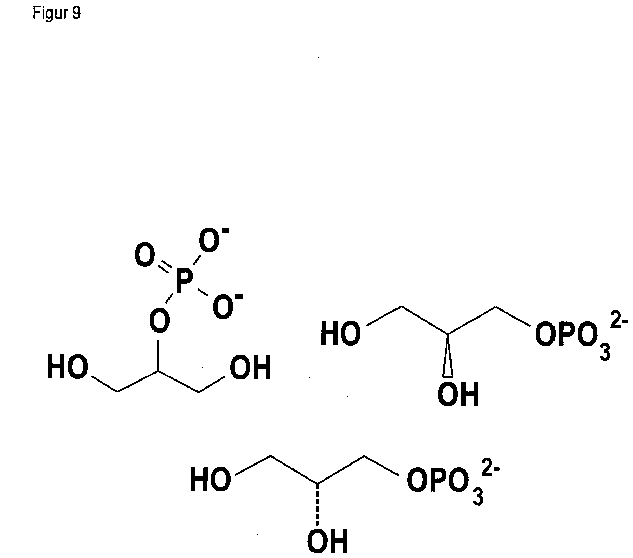

Dies wird im Folgenden anhand der Zugabe einer Mischung von Glycerin-2-*ortho-*phosphat und Glycerin-3-ortho-phosphat (im Folgenden einfach "*Glycerin-ortho-phosphat*") zu einer Dialyselösung veranschaulicht. Bei dieser Substanz handelt es sich um einen Vertreter der organischen Ester der Phosphorsäure. Figur 9 zeigt die Strukturformeln des Glycerin-2-ortho-phosphats und des Glycerin-3-ortho-phosphats.

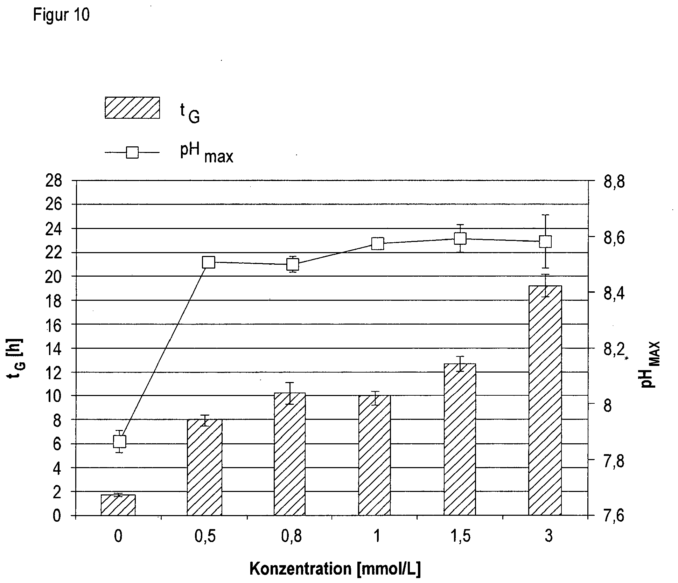

In Figur 10 wird die Stabilität mehrerer mit unterschiedlichen Konzentrationen an Glycerin-ortho-phosphat versetzten "*multiBic*"-Lösungen dargestellt. Wie daraus ersichtlich ist, steigen mit zunehmender Konzentration an Glycerin-*ortho*-phosphat sowohl pHₘₐₓ als auch die t_{G}-Werte der Lösung an. Auffallend ist dabei, dass der pHₘₐₓ bereits bei einer Konzentration von 0,5 mmol/l deutlich gegenüber einer herkömmlichen "*multiBic*"-Lösung erhöht ist. Auch im physiologisch relevanten Bereich von 0,8 bis 1,25 mmol/l erfährt die Lösung einen deutlichen Stabilitätsgewinn.

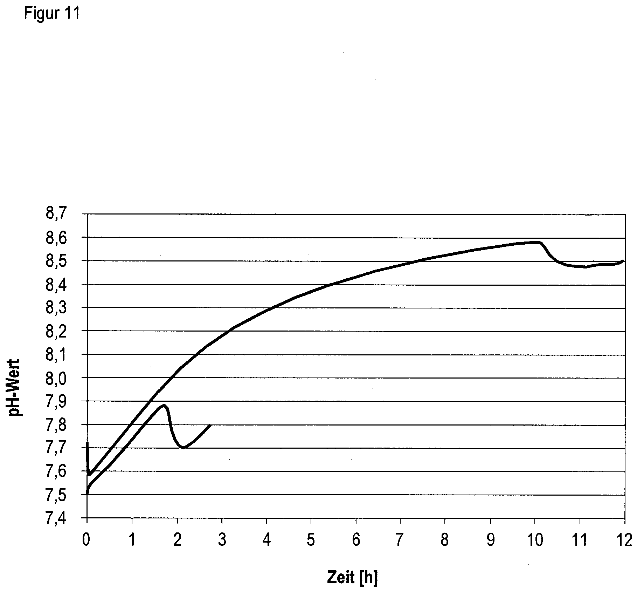

Des Weiteren deutet der in Figur 11 gezeigte pH-Verlauf darauf hin, dass im Falle einer Ausfällung bei hohen pH-Werten (obere Kurve) nur-Calciumcarbonat als schwerlösliche Verbindung entsteht, da der typische Kurvenverlauf mit signifikantem Abfallen des pH-Wertes beim Eintritt der Fällung zu erkennen ist (analog zu einer herkömmlichen "*multiBic*"-Lösung, untere Kurve).

Diese Annahme kann durch eine Bestimmung des Phosphatgehaltes mittels UV-vis Spektroskopie (enzymatisches Test-Kit) auch bestätigt werden.

Vergleicht man die in der nachfolgenden Tabelle 5 dargestellten Werte einer P-modifizierten "*multiBic*"-Lösung gemäß Vergleichsbeispiel mit den Werten einer erfindungsgemäß mit 1 mmol/l Glycerin-ortho-phosphat ("*GP*") versetzten "*multiBic"-*Lösung, so sieht man, dass bei der P-modifizierten "*multiBic"*-Lösung nach der Fällung die Phosphatkonzentration nur noch -67% des Ausgangswertes beträgt, wohingegen bei der GP-modifizierten "*multiBic"-Lösung* der Phosphatgehalt unverändert bleibt.

**Tabelle 5:**

| | Phosphatgehalt vor Fällung [mg/l] | Phosphatgehalt nach Fällung [mg/l] |
|---|---|---|
| P-modifizierte Lösung | 30 | 21 |
| GP-modifizierte Lösung | 22* | 22* |

| | | |
|---|---|---|
| die Absolutwerte der Messung weichen vom wahren Wert ab, da kein für Glycennortho-phosphat spezifisches Testkit verwendet wurde, sondern mit dem Testkit für ortho-phosphat gearbeitet wurde | | |

Ein Vergleich der pHₘₐₓ-Werte der GP-modifizierten "*multiBic*"-Lösung mit den *ortho-*Phosphat-haltigen Lösungen des Vergleichsbeispiels zeigt weiterhin, dass der pHₘₐₓ-Wert der Lösung mit Glycerin-ortho-Phosphat am höchsten ist (Tabelle 6).

**Tabelle 6**

| | pHmax | Ausfällungsprodukt |
|---|---|---|
| unmodifizierte "*multiBic*"-Lösung | 7,9 | Calciumcarbonat |
| P-modifizierte "*multiBic "-Lösung* | 8,3* | Calciumphosphat |
| "*multiPlus*"-Lösung . | 8,3* | Calciumphosphat |
| *"Phoxilium"-Lösung* | 8,2* | Calciumphosphat |
| GP-modifizierte "*multiBic*"-Lösung | 8,6 | Calciumcarbonat |

| | | |
|---|---|---|
| * abgeleitet aus Trübungs- und/oder Partikelmessung | | |

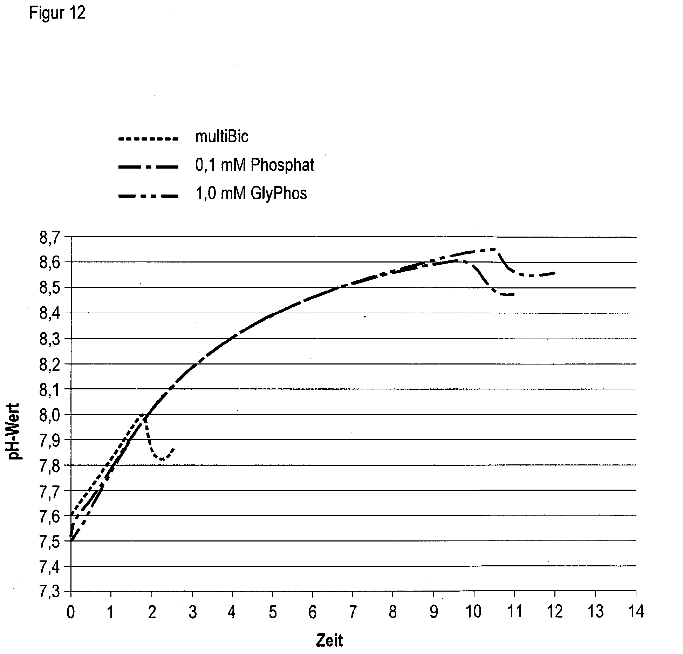

Figur 12 zeigt einen Vergleich der stabilisierenden Wirkung von Glycerin-ortho-phosphat im physiologischen Konzentrationsbereich mit der von ortho-Phosphat. Wie daraus ersichtlich ist, bringt der Zusatz von 1,0 mmol/l Glycerin-ortho-phosphat zur "*multiBic*"-Lösung nahezu den gleichen stabilisierenden Effekt wie der Zusatz von 0,1 mmol/l ortho-Phosphat. Weiterhin werden beim Zusatz von Glycerin-ortho-phosphat keine schwerlöslich Phosphate als Fällungsprodukte gebildet.

Durch Zusatz von Glycerin-ortho-phosphat kann somit die benötigte physiologische Konzentration an Phosphat in einer Dialyselösung bereitgestellt werden und gleichzeitig die Stabilität im Vergleich zu phosphatfreien oder ortho-Phosphat-haltigen Lösungen signifikant erhöht werden. Somit wird durch diese Rezeptur eine Dialyselösung, vorzugsweise eine HF/HD-Lösung mit physiologischer Phosphatkonzentration erhalten die über eine verbesserte Haltbarkeitsdauer verfügt und über einen Zeitraum von z.B. 24 Monaten sicher anzuwenden ist.

### Ausführungsbeispiel 2

Zur GP-modifizierten *"multiBic"-Lösung* aus Ausführungsbeispiel 1 wird ferner *ortho-*Phosphat zugegeben.

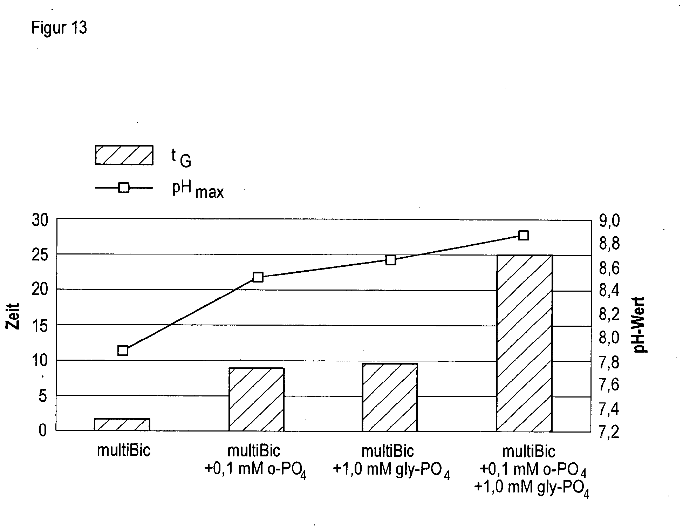

In Figur 13 wird die Stabilität mehrerer der folgenden Lösungen dargestellt: unmodifizierte "*multiBic*"-Lösung gemäß Vergleichsbeispiel, P-modifizierte "*multiBic"-*Lösung gemäß Vergleichsbeispiel, GP-modifizierte *"multiBic"-Lösung* gemäß Ausführungsbeispiel 1 und eine zusätzlich mit 0,1 mmol/l an ortho-Phosphat versetzte GP-modifizierten "*multiBic"-Lösungen.* Wie aus der Figur ersichtlich ist, ergibt sich für die sowohl mit Glycerin-ortho-phosphat als auch mit ortho-Phosphat versetzte Lösung eine überproportionale Steigerung der Stabilität gegenüber der P-modifizierten und der GP-modifizierten "*multiBic*"-Lösung.

Die Werte wurden wie oben beschrieben anhand der "*Rapid Controlled Precipitation"-Methode* bei T=40°C mit pH-Messung bzw. sofern nicht anwendbar mit Trübungs- und/oder Partikelmessung als Detektion erhalten.

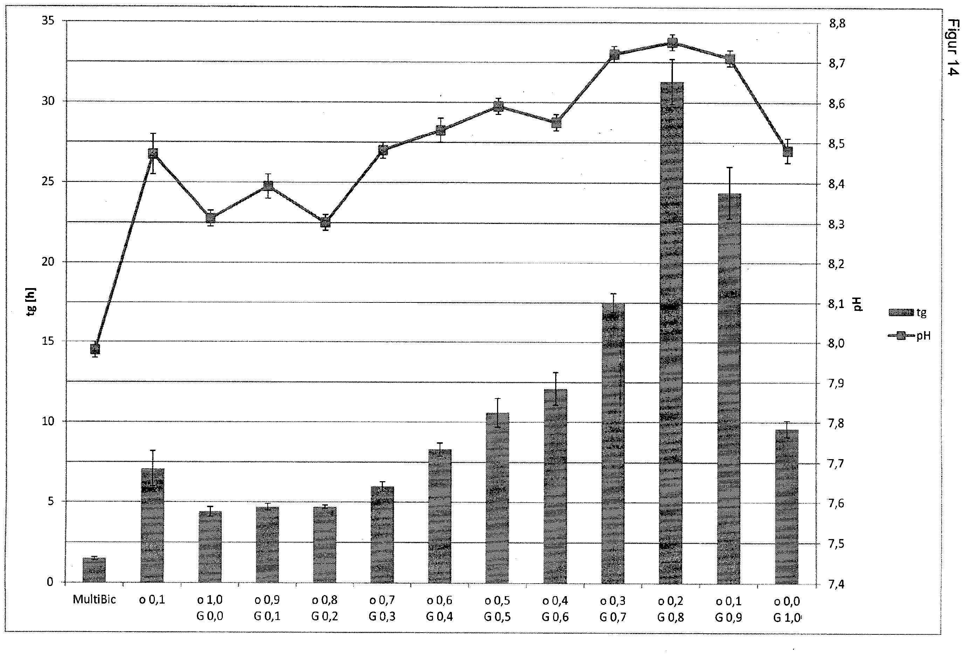

Figur 14 zeigt in Form der zwei links dargestellten Messpunkte zum einen die Stabilität einer unmodifizierten "*multiBic*"-Lösung sowie einer *ortho*-Phosphat haltigen Dialyselösung, jeweils ohne Glycerin-*ortho*-phosphat. Die Lösungstemperatur betrug 40 °C.

Die weiteren Messungen betreffen Dialyselösungen, die eine Mischung aus Glycerin-*ortho*-phosphat mit ortho-Phosphat enthalten. Die Konzentration (jeweils in mmol/l) von ortho-Phosphat ist neben dem Bezugszeichen "o" und die Konzentration von Glycerin-ortho-Phosphat ist neben dem Bezugszeichen "G" angegeben. Wie dies aus Figur 14 ersichtlich ist, beträgt die Gesamtkonzentration beider Substanzen in der Dialyselösung stets 1,0 mmol/l.

Wie dies aus Figur 14 hervorgeht, werden besonders stabile Lösungen insbesondere bei Mischungs- bzw. Konzentrationsverhältnissen von Glycerin-ortho-Phosphat/ortho-Phosphat zwischen 0,7/0,3 und 0,9/0,1 erhalten.

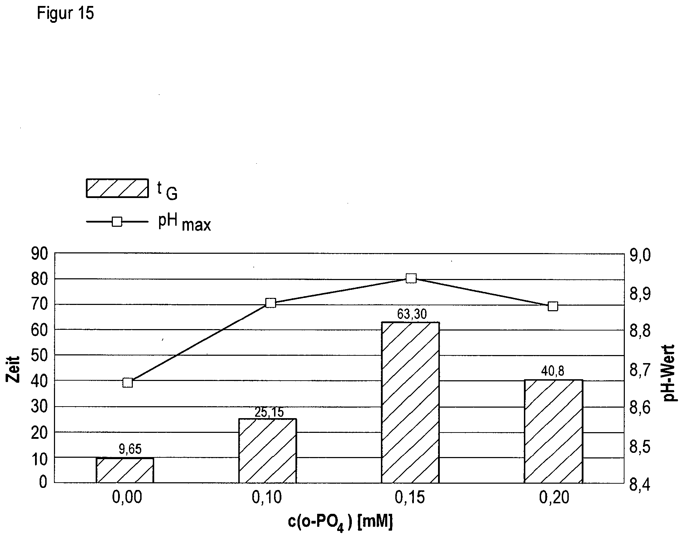

Figur 15 zeigt die Stabilität mehrerer zusätzlich mit unterschiedlichen Konzentrationen an *ortho*-Phosphat versetzten GP-modifizierten "*multiBic"-*Lösungen. Wie daraus ersichtlich ist, wird ein Stabilitätsmaximum korrespondierend zu einem maximalen pHₘₐₓ-Wert bei einer Zugabe von 0,15 mmol/l ortho-Phosphat erreicht.

### Fazit aus den Beispielen

Die Zugabe von Glycerin-*ortho*-phosphat in physiologischen Konzentrationsbereichen, führt zu einer signifikanten Stabilisierung von Calcium- und/oder Magnesium-Ionenhaltigen Bicarbonat-gepufferten Dialyselösungen. Ausfällungsreaktionen können hierdurch bis zu pH-Werten pH>8 vermieden werden, was die Sicherheit als auch die Haltbarkeit von Dialyselösungen deutlich verbessert. Ein wesentlicher Aspekt der vorliegenden Erfindung besteht darin, dass die organischen Ester der Phosphorsäure sowohl als *in-use* Stabilisierungs-Agens als auch in medizinisch relevanter Konzentration als Phosphatquelle zur Regulation des Phosphathaushaltes fungieren können. Ein Vorteil gegenüber handelsüblichen Dialyselösungen besteht in der weitaus höheren Stabilität der Lösung gegenüber Ausfällungsreaktionen von Calciumcarbonat (erhöhte pHₘₐₓ-Werte und respektive t_{G}-Zeiten). Eine Ausfällung von Calciumphosphat wird im Gegensatz zu den sich bereits auf dem Markt befindlichen ortho-Phosphat-haltigen Lösungen vermieden.

Durch die die Zugabe Glycerin-ortho-phosphat und ferner *ortho-*Phosphat kann sich ein synergischer Effekt einstellen, der die beschriebenen Wirkungen des organischen Esters der Phosphorsäure noch verbessert.

## Patentansprüche

1. Dialyselösung oder Substitutionslösung enthaltend Bicarbonat-Ionen sowie Calcium- und/oder Magnesium-Ionen, wobei die Lösung Natrium-Ionen, Kalium-lonen und/oder Chlorid-Ionen als weitere Elektrolyte enthält,
**dadurch gekennzeichnet,**
**dass** die Dialyselösung oder die Substitutionslösung Glycerin-ortho-phosphat und ferner ortho-Phosphat enthält, wobei die Dialyselösung oder Substitutionslösung veresterte und nicht veresterte Phosphatgruppen in der Summe in einer Konzentration von 0,8 bis 1,25 mmol/l enthält.

2. Dialyselösung oder Substitutionslösung nach Anspruch 1, **dadurch gekennzeichnet, dass** die Dialyselösung oder die Substitutionslösung das Glycerin-ortho-phosphat in einer Konzentration von 1 bis 1,2 mmol/l enthält.

3. Dialyselösung oder Substitutionslösung nach einem der vorhergehenden Ansprüche, **dadurch gekennzeichnet, dass** das Konzentrationsverhältnis zwischen dem Glycerin-ortho-phosphat und dem ortho-Phosphat in der Spanne zwischen 0,3/0,7 und 0,9/0,1 liegt.

4. Dialyselösung oder Substitutionslösung nach einem der vorhergehenden Ansprüche, **dadurch gekennzeichnet, dass** die Dialyselösung oder die Substitutionslösung ferner ein Osmotikum, vorzugsweise ein Saccharid, insbesondere Glukose, enthält.

5. Kombination aus mehreren, vorzugsweise aus genau zwei Einzellösungen, die derart ausgebildet sind, dass sie nach ihrem Mischen miteinander eine Dialyselösung oder Substitutionslösung gemäß einem der vorhergehenden Ansprüche ausbilden, wobei die mehreren, vorzugsweise genau zwei Einzellösungen, in einem Mehrkammerbeutel vorliegen, welcher wenigstens zwei Kammern umfasst, wobei eine der Kammern eine erste Einzellösung der Kombination und eine andere Kammer eine zweite Einzellösung der Kombination aufweist.

6. Kombination nach Anspruch 5, **dadurch gekennzeichnet, dass** nur eine der Einzellösungen den organischen Ester der Phosphorsäure und eine *ortho*Phosphorsäure enthält.

7. Kombination nach einem der Ansprüche 5 bis 6, **dadurch gekennzeichnet, dass** eine erste Einzellösung Calcium- und/oder Magnesium-Ionen enthält und eine zweite Einzellösung, die keine Calcium- und/oder Magnesium-Ionen enthält, den organischen Ester der Phosphorsäure und die ortho-Phosphorsäure enthält.

8. Kombination nach einem der Ansprüche 5 bis 7, **dadurch gekennzeichnet, dass** eine erste Einzellösung Calcium- und/oder Magnesium-Ionen, Chlorid-Ionen, ein Osmotikum und ggf. Kalium-Ionen enthält und eine zweite Einzellösung Natrium-Ionen, Chlorid-Ionen, Bicarbonat-Ionen, den organischen Ester der Phosphorsäure und die ortho-Phosphorsäure enthält.

9. Kombination nach Anspruch 8, **dadurch gekennzeichnet, dass** die erste Einzellösung keine Bicarbonat-Ionen und/oder keinen organischen Ester der Phosphorsäure und/oder keine ortho-Phosphorsäure und/oder keine Natrium-Ionen enthält.

10. Kombination nach einem der Ansprüche 8 bis 9, **dadurch gekennzeichnet, dass** die zweite Einzellösung keine Calcium- und/oder Magnesium-Ionen und/oder keine Kalium-Ionen und/oder kein Osmotikum enthält.

11. Kombination nach einem der Ansprüche 5 bis 10, **dadurch gekennzeichnet, dass** eine erste Einzellösung einen pH-Wert im Bereich von 2,4 bis 3,0 aufweist und dass eine zweite, das Glycerin-ortho-phosphat enthaltende Einzellösung einen pH-Wert im Bereich von 7,0 bis 7,8 aufweist.

## Claims

1. Dialysis solution or a substitution solution containing bicarbonate ions as well as calcium ions and/or magnesium ions, wherein the solution contains sodium ions, potassium ions and/or chloride ions as further electrolytes,
**characterized in that**
the dialysis solution or the substitution solution contains glycerol orthophosphate and also orthophosphate, wherein the dialysis solution or substitution solution contains esterified or non-esterified phosphate groups in the sum of a concentration of 0.8 to 1.25 mmol/l.

2. Dialysis solution or a substitution solution in accordance with claim 1, **characterized in that** the dialysis solution or the substitution solution contains the glycerol orthophosphate in a concentration of 1 to 1.2 mmol/l.

3. Dialysis solution or a substitution solution in accordance with one of the preceding claims, **characterized in that** the concentration ratio between the glycerol orthophosphate and the orthophosphate is in the range between 0.3/0.7 and 0.9/0.1.

4. Dialysis solution or a substitution solution in accordance with one of the preceding claims, **characterized in that** the dialysis solution or the substitution solution further contains an osmotic agent, preferably a saccharide, in particular glucose.

5. Combination of several, preferably of exactly two, individual solutions which are configured such that they form a dialysis solution or the substitution solution in accordance with one of the preceding claims after their mixing with one another, wherein the several, preferably of exactly two, individual solutions are present in a multi-chamber bag comprising at least two chambers, wherein one of the chambers has a first individual solution of the combination and another chamber has a second individual solution of the combination.

6. Combination in accordance with claim 5, **characterized in that** only one of the individual solutions contains the organic ester of phosphoric acid and the orthophosphoric acid.

7. Combination in accordance with one of the claims 5 to 6, **characterized in that** a first individual solution contains calcium ions and/or magnesium ions and a second individual solution which does not contain any calcium ions and/or magnesium ions contains the organic ester of phosphoric acid and the orthophosphoric acid.

8. Combination in accordance with one of the claims 5 to 7, **characterized in that** a first individual solution contains calcium ions and/or magnesium ions, chloride ions, an osmotic agent and optionally potassium ions and a second individual solution contains sodium ions, chloride ions, bicarbonate ions, the organic ester of phosphoric acid and the orthophosphoric acid.

9. Combination in accordance with claim 8, **characterized in that** the first individual solution contains no bicarbonate ions and/or no organic ester of phosphoric acid and/or no orthophosphoric acid and/or no sodium ions.

10. Combination in accordance with one of the claims 8 to 9, **characterized in that** the second individual solution contains no calcium ions and/or magnesium ions and/or no potassium ions and/or no osmotic agent.

11. Combination in accordance with one of the claims 5 to 10, **characterized in that** a first individual solution has a pH in the range from 2.4 to 3.0; and **in that** a second individual solution containing the glycerol orthophosphate has a pH in the range from 7.0 to 7.8.

## Revendications

1. Solution de dialyse ou solution de substitution contenant des ions bicarbonate ainsi que des ions calcium et/ou magnésium, la solution contenant des ions sodium, des ions potassium et/ou des ions chlorure comme autres électrolytes,
**caractérisée en ce que**
la solution de dialyse ou la solution de substitution contient du glycérol-orthophosphate et en outre de l'orthophosphate, la solution de dialyse ou la solution de substitution contenant des groupes phosphate estérifiés et non estérifiés au total dans une concentration de 0,8 à 1,25 mmol/l.

2. Solution de dialyse ou solution de substitution selon la revendication 1, **caractérisée en ce que** la solution de dialyse ou la solution de substitution contient le glycérol-orthophosphate dans une concentration de 1 à 1,2 mmol/l.

3. Solution de dialyse ou solution de substitution selon l'une des revendications précédentes, **caractérisée en ce que** le rapport de concentration entre le glycérol-orthophosphate et l'orthophosphate est compris dans l'intervalle de 0,3/0,7 à 0,9/0,1.

4. Solution de dialyse ou solution de substitution selon l'une des revendications précédentes, **caractérisée en ce que** la solution de dialyse ou la solution de substitution contient en outre un osmotique, de préférence un saccharide, en particulier du glucose.

5. Combinaison de plusieurs, de préférence d'exactement deux solutions individuelles, qui sont conçues de telle manière qu'après avoir été mélangées les unes aux autres, elles forment une solution de dialyse ou une solution de substitution selon l'une des revendications précédentes, les plusieurs, de préférence exactement deux solutions individuelles se présentant dans une poche à compartiments multiples qui comprend au moins deux compartiments, l'un des compartiments présentant une première solution individuelle de la combinaison et un autre compartiment une seconde solution individuelle de la combinaison.

6. Combinaison selon la revendication 5, **caractérisée en ce qu'**une seule des solutions individuelles contient l'ester organique de l'acide phosphorique et un acide orthophosphorique.

7. Combinaison selon l'une des revendications 5 à 6, **caractérisée en ce qu'**une première solution individuelle contient des ions calcium et/ou magnésium et une seconde solution individuelle, qui ne contient pas d'ions calcium et/ou magnésium, contient l'ester organique de l'acide phosphorique et l'acide orthophosphorique.

8. Combinaison selon l'une des revendications 5 à 7, **caractérisée en ce qu'**une première solution individuelle contient des ions calcium et/ou magnésium, des ions chlorure, un osmotique et le cas échéant des ions potassium et une seconde solution individuelle contient des ions sodium, des ions chlorure, des ions bicarbonate, l'ester organique de l'acide phosphorique et l'acide orthophosphorique.

9. Combinaison selon la revendication 8, **caractérisée en ce que** la première solution individuelle ne contient pas d'ions bicarbonate et/ou pas d'ester organique de l'acide phosphorique et/ou pas d'acide orthophosphorique et/ou pas d'ions sodium.

10. Combinaison selon l'une des revendications 8 à 9, **caractérisée en ce que** la seconde solution individuelle ne contient pas d'ions calcium et/ou magnésium et/ou pas d'ions potassium et/ou pas d'osmotique.

11. Combinaison selon l'une des revendications 5 à 10, **caractérisée en ce qu'**une première solution individuelle présente une valeur de pH comprise dans la plage de 2,4 à 3,0 et **en ce qu'**une seconde solution individuelle, contenant le glycérol-orthophosphate, présente une valeur de pH comprise dans la plage de 7,0 à 7,8.
